# EUROPEAN PATENT APPLICATION

(11) **EP 4 674 420 A1**
(43) Date of publication of application: **07.01.2026**
(21) Application number: 24382717.7
(22) Date of filing: 03.07.2024
(51) Int. Cl.: A61K 35/57, A23L 15/00, A61K 38/48, A61Q 19/00, A61P 19/00

(54) **COMPOSITION AND METHOD FOR OBTAINING EGGSHELL MEMBRANE PRODUCTS SUITABLE FOR ORAL AND TOPICAL USE**

(71) Applicant: Eggnovo S.L., 31132 Villatuerta (Navarra) (ES)
(72) Inventor: AGUIRRE GONZÁLEZ, Andrés, 31192 Mutilva, Navarra (ES); GIL QUINTANA, Erena, 31620 Huarte, Navarra (ES); OYEREGUI SOLA, Peio, 31200 Estella, Navarra (ES); NIETO IRIBERRI, Oihane, 31200 Estella, Navarra (ES); GONZÁLEZ DEL POZO, Alicia, 31180 Zizur Mayor, Navarra (ES)
(74) Representative: Elzaburu S.L.P.

(57) **Abstract**

The present invention relates to a composition and method for solubilizing eggshell membrane at convenient rates. The present invention also relates to organoleptically acceptable products obtained therein, and to their oral or topical application to improve a disease or condition in a subject.

## Description

### FIELD OF THE INVENTION

The present invention relates to composition and method for obtaining solubilized eggshell membrane products. The present invention also relates to the solubilized eggshell membrane products obtained by the method of the present invention, to compositions comprising them, as well as to the use of products obtained therein for oral and topical use.

### BACKGROUND OF THE INVENTION

The use of eggshell membrane has demonstrated its effectiveness in improving joint and skin health. Its great insolubility and low digestibility rate, however, limit its use to oral applications since it requires the hydrolysis that occurs in the gastro-intestinal tract to generate active peptides that have an effect on different target cells of the body. Due to the structural complexity of the membrane, it is not fully digested after ingestion. As shown in different studies, only 45-50% of the egg membrane is digested in the stomach, the remaining 50-55% is evacuated from the stomach without being digested (Jia, H. et al. 2017, Sci. Rep. 7, 43993).

The possibility of exogenously hydrolysing the eggshell membrane can be an effective method to increase both its hydrolysis rate and solubility, and thus its effectiveness.

Different technologies to exogenously hydrolyse the membrane have been described.

These include chemical methods, which make use of both strong acids and bases (eg. US 2010/0029564A1), and enzymatic methods, which consist of membrane hydrolysis by means of proteolytic enzymes (EP3653721B1, EP2612922A1). Enzymatic hydrolysis displays several advantages when compared with chemical hydrolysis, including high specificity and repeatability. Due to the specificity of enzymes, the result achieved is always the same, as long as the optimal temperature and pH conditions for the enzyme used are maintained.

The hydrolysis methods that exist today are mainly focused on solving the technological challenge of membrane water insolubility. In many cases, the products obtained by such existing methods are not suitable for being used in the areas of food and cosmetics. Some of the reasons being (i) that the reagents used are considered allergens, and are thereby not valued in these sectors, (ii) the partial hydrolysis rates achieved, which mean very low productivity levels and therefore low economic interest, and (iii) the fact that the final products obtained display organoleptic characteristics of smell and taste that are not acceptable to the end consumer, this constituting a drawback for their use.

The systems available so far for producing hydrolysis of the eggshell membrane make use of reagents which either are not suitable for human consumption or which, despite being usable, provide undesirable odour and taste properties that make the final product obtained not organoleptically acceptable. Some of the most commonly used reagents for eggshell solubilization are sulphites, e.g. metabisulphite, which bring an unpleasant sulphurous taste and smell to food products, and also cause degradation of certain compounds in the food, negatively affecting the taste, aroma and colour of the final product. In other cases, the reagents used can cause allergies and intolerances, their actual use thus being limited by this fact, or directly forbidden (eg. DTT). Use of any such products also faces regulatory problems.

There is thus, the technical problem to provide for eggshell hydrolysis methods and reagents which overcome the drawbacks of existing methods mentioned above, while maintaining an adequate hydrolysis rate of the eggshell membrane.

It would be desirable that the products obtained from this method overcome the organoleptic and allergenic drawbacks of existing products, and be suitable for being included in products that can be ingested by human an animal subjects (fortified foods, food supplements, drinkable supplements) as well as in products that can be applied topically to these subjects (creams, serums, lotions).

### SUMMARY OF THE INVENTION

The present invention overcomes the problems of the state of the art, by means of the aspects and embodiments described below.

A first aspect of the present invention relates to a composition for solubilizing eggshell membrane characterized in that it comprises a cysteine protease and L-cysteine HCl Anhydrous as reducing agent. Preferably, the cysteine protease is papain. For concentrations of papain and/ or L-cysteine HCl Anhydrous higher than 10 g/100 g eggshell membrane, a masking agent may also be included.

A second aspect of the present invention relates to a method for solubilizing eggshell membrane, comprising a hydrolysis phase wherein the eggshell membrane is treated with a composition which comprises a cysteine protease and L-cysteine HCl Anhydrous as reducing agent.

Preferably, the hydrolysis phase may be followed by a filtration phase, a concentration phase and a drying phase.

Preferably, the cysteine protease is papain in a concentration of at least 0.1 g/ 100 g eggshell membrane. Most preferably, the cysteine protease is papain in a concentration in the range of 0.1 g/ 100 g eggshell membrane to 50 g/ 100 g eggshell membrane, most preferably in a concentration in the range of 0.1 g/ 100 g eggshell membrane to 10 g/ 100 g eggshell membrane, and even most preferably, papain in a concentration in the range of 2 g/ 100 g eggshell membrane to 6 g/ 100 g eggshell membrane.

Preferably, the L-cysteine HCl Anhydrous concentration is in the range of 0.5 g/ 100 g eggshell membrane to 50 g/ 100 g eggshell membrane. Most preferably, in the range of 0.5 g/ 100 g eggshell membrane to 10 g/ 100 g eggshell membrane.

For concentrations of papain and/ or L-cysteine HCl Anhydrous higher than 10 g/ 100 g eggshell membrane, a masking agent may also be included.

A third and fourth aspect of the present invention relate, respectively, to the solubilized eggshell membrane products obtained by the eggshell membrane solubilization method of the invention, as well as to a composition comprising the solubilized eggshell membrane products therein.

A fifth aspect of the present invention relates to the use of the composition of aspect four as a medicine, quasi drug, food, or cosmetic product, dispensed in the form of products for topical use, including creams, serums, lotions, shampoos, conditioners, nail polish, etc, or in the form of products for oral use, including supplements or functional foods, as drinkable or edible products.

Use of this composition has proved useful to various applications, and in particular for improving skin, hair and nail health and/or gastrointestinal and joint condition or disease of a subject, in particular symptoms produced by osteoarthrosis and osteoarthritis.

Additional aspects of the present invention relate to products for topical use, including creams, serums, lotions, shampoos, conditioners, nail polish, etc., as well as to products for oral use, including drinkable and edible products comprising the solubilized eggshell membrane products of the present invention, as well as to particular methods to respectively produce the topical and oral use products.

The inventors have surprisingly identified that making use of a composition of cysteine protease and L-Cysteine HCl Anhydrous as reducing agent for eggshell membrane solubilization, results in bioactive peptides of better quality, and improved odour, colour and taste, than those obtained with compositions of different reducing agents (including other reducing agents acceptable for the food industry, such as non-leavening yeast, L-cysteine, N-acetyl cysteine).

In addition, hydrolysis of eggshell membrane with the composition of cysteine protease and L-Cysteine HCl Anhydrous of the present invention, displays a hydrolysis rate which is equivalent to the highest rates available (eg. Metabisulphite) and definitely more advantageous than those corresponding to other reducing agents acceptable for the food industry.

The inventors have additionally devised a method for solubilizing eggshell membranes, of which the first step is the hydrolysis reaction with the composition of cysteine protease and L-Cysteine HCl Anhydrous. This hydrolysis step is compatible with subsequent steps including a filtration phase, a concentration phase and a drying phase, and results in products of acceptable organoleptic (including colour, odour and taste) properties, that can be used in both oral and topical products without concentration limitations.

Several advantages of the method of the present invention include that differently to other methods of the state of the art, the hydrolysis rate of the method of the present invention is very high despite using natural reagents accepted in both the food and cosmetic sectors. This implies a very affordable industrial cost.

Also, the yield of biomolecules obtained is also higher than that of other methods that include additional purification steps wherein part of the biomolecules is lost.

Particular administration means of the products obtained by the method of the present invention include but are not limited to products that can be ingested (functional and fortified foods, food supplements, functional beverages, juices, etc.), as well as products that can be applied topically (cosmetic products, creams, serums, lotions, shampoos, conditioners, nail polish, etc.).

Main uses of the products of the present invention relate to their activity as a collagen, elastin and hyaluronic acid booster, both for increasing their neogenesis and for slowing down their degradation, as well as to their anti-inflammatory, antioxidant and healing activity.

### BRIEF DESCRIPTION OF FIGURES

**Figure 1****:** Results and graph showing ROS production of human HeLa cell line *in vitro* in the presence of 50 µM Antimycin, with the hydrolysate in the concentration 0.0120% and 0.0084%.
**Figure 2****:** Results and images showing results of the healing test of the hydrolysate in the concentration of 0.0084% on adult human keratinocytes *in vitro.*
**Figure 3****:** Results of the effectiveness test of creams of the present invention, carried out on 60 individuals divided into three groups of 20 individuals. Group A tested cream nº2, group B tested cream nº4 and group C tested cream nº6, for 60 days.
**Figure 4****:** Results of organoleptic properties of the creams of the present invention.

### DESCRIPTION OF THE INVENTION

The basis of the present invention is the use of a composition comprising a cysteine protease as protease, and L-cysteine HCl Anhydrous as reducing agent, for solubilizing eggshell membranes.

In a preferred embodiment, cysteine protease may be made use of, preferably papain, bromelain, ficine, pepsin and protease K, and most preferably, papain.

The protease used in the present invention is not particularly limited as long as it achieves efficient solubilization of eggshell membranes. The protease may be a commercially available enzyme preparation.

Preferably, the cysteine protease is papain in a concentration of at least 0.1 g/ 100 g eggshell membrane. Most preferably, the cysteine protease is papain in a concentration in the range of 0.1 g/ 100 g eggshell membrane to 50 g/ 100 g eggshell membrane, most preferably in a concentration in the range of 0.1 g/ 100 g eggshell membrane to 10 g/ 100 g eggshell membrane, and even most preferably, papain in a concentration in the range of 2 g/ 100 g eggshell membrane to 6 g/ 100 g eggshell membrane.

When concentrations of papain lower than the minimum 0.1 g/ 100 g eggshell membrane value are used, the hydrolysis rates achieved are not satisfactory. Contrarily, any concentration of papain equal to or higher than 0.1 g/ 100 g eggshell membrane, prove to achieve adequate hydrolysis rates. For concentrations of papain higher than 10 g/ 100 g eggshell membrane, a masking agent may also be included.

Preferably, the L-cysteine HCl Anhydrous concentration is in the range of 0.5 g/ 100 g eggshell membrane to 50 g/ 100 g eggshell membrane. Most preferably, in the range of 0.5 g/ 100 g eggshell membrane to 10 g/ 100 g eggshell membrane. For concentrations of L-cysteine HCl Anhydrous higher than 10 g/ 100 g eggshell membrane, a masking agent may also be included.

A preferred composition of the present invention comprises papain in a concentration of at least 0.1 g/ 100 g eggshell membrane, and L-cysteine HCl Anhydrous concentration in the range of 0.5 g/ 100 g eggshell membrane to 50 g/ 100 g eggshell membrane. Preferably, papain in a concentration in the range of 0.1 g/ 100 g eggshell membrane to 50 g/ 100 g eggshell membrane, and L-cysteine HCl Anhydrous concentration in the range of 0.5 g/ 100 g eggshell membrane to 50 g/ 100 g eggshell membrane. Most preferably, the composition comprises papain in a concentration in the range of 0.1 g/ 100 g eggshell membrane to 50 g/ 100 g eggshell membrane, and L-cysteine HCl Anhydrous concentration in the range of 0.5 g/ 100 g eggshell membrane to 10 g/ 100 g eggshell membrane. And even most preferably, papain in a concentration in the range of 2 g/ 100 g eggshell membrane to 6 g/ 100 g eggshell membrane and L-cysteine HCl Anhydrous concentration in the range of 0.5 g/ 100 g eggshell membrane to 10 g/ 100 g eggshell membrane.

For concentrations of papain and/ or L-cysteine HCl Anhydrous higher than 10 g/ 100 g eggshell membrane, a masking agent may also be included to ameliorate the organoleptic acceptance of the composition.

As masking agent it is understood any agent with ability to mask undesirable flavours and/ or odours. Masking agents of the present invention include, but are not limited to:

| **Masking agent** | **Concentration range** |
|---|---|
| Lemon | 5-15 % |
| Grapefruit | 10-15 % |
| Strawberry | 20-25 % |
| Pineapple | 10-15 % |
| Vanilla | 10-15 % |
| Banana | 15-20 % |
| Raspberry | 20-30% |
| Cofee | 25-50 % |

In the present description, "eggshell membrane" or "membrane" means the membrane located at the inside of the outer shell of eggs of birds such as chicken, partridge, silky fowl, duck, goose, and ostrich. In the solubilization method of the present invention, eggshell membranes are solubilized through the treatment using the combination of a protease and a reducing agent. The state of the eggshell membranes to be treated is not particularly limited. For example, dry eggshell membranes prepared by separating the membranes from the outer shell, followed by drying (for example, solar drying, hot-air drying, vacuum drying, aspiration drying, or freeze drying), wet eggshell membranes before drying, or wet eggshell membranes prepared by swelling after drying may be used. Alternatively, the eggshell membranes may be shredded or pulverized (for example, powder). Yet alternatively, the eggshell membranes unseparated from the shell may be used.

The advantageous hydrolysis rates and organoleptically acceptable hydrolysis products of the present invention are equally obtained both when the starting raw material is eggshell and egg membrane previously separated from the shell.

Both possibilities are technologically feasible, each with its advantages and disadvantages. The major advantage of using eggshell as the starting raw material is that there is no need for a membrane separation line, but on the other hand, because approximately 97% of the eggshell is mineral material, mainly calcium carbonate, the tank where hydrolysis takes place requires a size 100 times larger, as well as a system to recover the non-hydrolysed solids, which is not necessary in the case of egg membrane separation, since a centrifugation system is more than enough to separate the non-digested solid material from the hydrolysate.

A second aspect of the present invention, relates to a method for solubilizing eggshell membrane, comprising a hydrolysis phase wherein the eggshell membrane is treated with the composition of the present invention.

The method of the present invention includes a hydrolysis step wherein a composition comprising a cysteine protease as protease, and L-cysteine HCl Anhydrous as reducing agent, is used for hydrolysing eggshell membranes.

In a preferred embodiment of the method of the present invention, the hydrolysis phase is followed by a filtration phase and/or a concentration phase and/or a drying phase. In the most preferred embodiment, the method of the present invention comprises a hydrolysis phase, a filtration phase, a concentration phase and a drying phase.

The method described herein is also useful for solubilizing eggshell membranes with compositions comprising a cysteine protease as protease, and an alternative reducing agent, preferably, Non-leavening yeast, L-cysteine or N-acetyl Cysteine.

In a preferred embodiment, the hydrolysis phase is performed at a temperature of between 30°C and 80°C; preferably, between 35 and 75; most preferably of between 45°C and 70ºC. Preferably, the reaction is carried out under neutral pH conditions. The neutral pH here means pH 6.0 to 8.0. In a preferred embodiment, the reaction is performed at pH adjusted to 6.5 to 7.5, most preferably, at pH adjusted to 7.0 to 7.5. In a preferred embodiment, the reaction time is established so as to achieve complete solubilization of eggshell membranes. Preferably, the reaction time is of minimum 2 hours for an adequate yield, with no particular maximum limit. Reaction times of between 4 and 24 hours, result in particularly good yields.

Preferably, the filtration phase consists of subjecting the hydrolysate to one or more of the following processes: centrifugation and/or decantation and/or filtration through ion exchange resins.

In another preferred embodiment, the concentration phase consists of evaporation and/or concentration by nanofiltration membranes.

Preferred techniques for the drying phase are one or more of freeze-drying, spray drying, drying in a fluid bed, or a combination thereof.

The method of the present invention proves equally efficient both for small scale reactions as well as at industrial scale.

The method of solubilization of eggshell membranes of the present invention has proven particularly useful at industrial scale, and comprises a hydrolysis phase, a filtration phase, a concentration phase and a drying phase.

Preferably, the hydrolysis of the industrial method takes place in a stainless steel tank, with a heating system and variable agitation system connected to a solids feed system and with a water inlet, which can either come directly from the mains or pass through a reverse osmosis filtration unit in order to be able to feed the system with osmosis water. Preferably, the heating system must be able to reach at least 90ºC, to allow proceed with inactivation of the enzyme in the reactor itself if necessary. Preferably, the reaction temperature is of between 30°C and 80°C; preferably, between 35 and 75; most preferably of between 45°C and 70ºC.

In a preferred embodiment, the filtration step of the industrial process consists of a phase separation system: decantation and/or centrifugation: Once the hydrolysis has been completed, the resulting product is subjected to a process of decantation or solid-liquid separation, so as to separate the liquid phase, which will contain the soluble material resulting from the hydrolysis, from the solid material, which will consist of both the fraction that has not been hydrolysed and the solid impurities such as remains of husk that may accompany the membrane powder. The feed tube is introduced into the bowl along its axis of rotation, and the lower part of the frame will have outlets for the solid phase at one end and for the liquid phase at the other. A casing protects the rotating parts, allowing the phases to be collected separately.

In another preferred embodiment, the filtration step of the industrial process consists of an Ion exchange resin filtration system. Most preferably, a phase consisting of a battery of three ion exchange resins of different types connected in series is incorporated into the process: A first cationic resin to remove cations, a second anionic resin to remove strong acid anions such as sulphates, nitrates and chlorides and a third adsorption resin to retain hydrophobic organic compounds.

This approach is most advantageous to obtain a product of high purity and with organoleptic characteristics that make it suitable for consumption in food and cosmetic products.

The industrial process of the present invention includes a concentration step, which allows concentrating the product before applying a drying system.

In a preferred embodiment, the concentration step has two concentration systems, which can be used either indistinctly or one first and then the other, also indistinctly:
Nanofiltration membrane system, which allows continuous operation, thus improving the efficiency and productivity of the concentration process; In addition to the low temperature required and the absence of severe conditions during operation prevents degradation of the peptides. This process is considered to be gentle as it does not damage the biological activity of the peptides, very selective as it allows the peptides to be separated from other components of smaller molecular size, such as water and salts, and is an energy efficient process.

On the other hand, the filtration flow rate can be slow and therefore prolong the processing time, low molecular weight peptides can be lost during the process, and a lot of maintenance and disinfection of the system is necessary to avoid membrane clogging, which could decrease the efficiency of the process, and increase the risk of microbiological contamination.

Vacuum evaporation system, which consists of lowering the pressure of the container containing the solution so that the temperature required for the evaporation of the solvent is lower than under atmospheric conditions. This method is particularly interesting as a system for concentrating a protein hydrolysate, since the low temperature required and the absence of oxygen help to prevent the degradation of the proteins during the concentration process.

On the other hand, it has a high energy consumption, and it is not a process that is easily controlled by the operators and, in addition, a large amount of foam is produced during the process that can drag part of the final product, reducing the productivity of the process.

Finally, the industrial process of the present invention includes a drying step, which includes one or more of the methods selected from freeze drying, spray drying and fluid bed drying.

In a preferred embodiment, the drying method is Freeze-drying: All the tests carried out at laboratory level have been obtained after drying the hydrolysate using freeze-drying as the drying method; additionally, the hydrolysate used in Example 5 for the preparation of the cream was also obtained by freeze-drying. This drying method is characterised by obtaining high quality products as the integrity of the heat-sensitive components is preserved, the final product was also shown to be highly soluble.

In another preferred embodiment, the drying method is spray drying: This drying method is the one used to obtain the material used in Example 6, which was used to produce a drinkable product with which the benefits at the joint level were tested. This drying system was shown to have high productivity, at a relatively low cost, resulting in a product in the form of a fine powder, whose particle size can be controlled by adjusting the process parameters.

In yet another preferred embodiment, the drying method is Fluidised bed drying: This drying system was used to obtain the material used in Example 4, in which the functional properties of the ingredients were tested through a battery of *in vitro* studies. This drying method proved to be relatively fast, allowing considerable amounts of product to be processed in a reasonable time, given that it is carried out at relatively low temperatures, reducing the risk of degradation of the heat-sensitive components of the hydrolysate. The longer residence time of the hydrolysate in contact with the hot air allows for uniform and consistent drying, which results in better solubility of the resulting product.

Additional aspects of the present invention relate to solubilized eggshell membrane products obtained by the eggshell membrane solubilization method, as well as to a composition containing the solubilized eggshell membrane products.

Yet additional aspects of the present invention relate to use of the composition of the invention as a medicine, quasi drug, food, or cosmetic product, dispensed in the form of products for topical use, in any possible galenic form, including for example creams, serums, lotions, shampoos, conditioners, nail polish, or in the form of products for oral use, including supplements or functional foods, as drinkable or edible products.

In a preferred embodiment, the composition is used to improve skin, hair and nail health and/or gastrointestinal or joint condition or disease of a subject, in particular symptoms produced by osteoarthrosis and osteoarthritis.

The application of the composition of the present invention is not particularly limited, and preferred applications include medicines, quasi drugs, foods, and cosmetics. More specifically, a preferred embodiment of the present invention provides a pharmaceutical composition, a quasi drug composition, a food composition, and a cosmetic composition containing solubilized eggshell membranes. Examples of the application and effect of the pharmaceutical composition and quasi drug composition of the present invention include prevention of oxidation, inhibition of growth of bacteria, prevention of inflammation, wound healing, blood pressure lowering, hair fostering, and nutritional supplementation.

The pharmaceutical composition and quasi drug composition of the present invention may be formulated in accordance with a common procedure. The formulation may contain pharmaceutically acceptable other components (for example, carriers, excipients, disintegrating agents, buffers, emulsifying agents, suspending agents, soothing agents, stabilizers, preservatives, antiseptics, and a normal saline solution). Examples of the excipient include lactose, starch, sorbitol, D-mannitol, and white sugar. Examples of the disintegrating agent include starch, carboxymethyl cellulose, and calcium carbonate. Examples of the buffer include phosphates, citrates, and acetates. Examples of the emulsifying agent include gum arabic, sodium alginate, and gum tragacanth. Examples of the suspending agent include glycerol monostearate, aluminum monostearate, methyl cellulose, carboxymethyl cellulose, hydroxymethyl cellulose, and sodium lauryl sulfate. Examples of the soothing agent include benzyl alcohol, chlorobutanol, and sorbitol. Examples of the stabilizer include propylene glycol and ascorbic acid. Examples of the preservative include phenol, benzalkonium chloride, benzyl alcohol, chlorobutanol, and methylparaben. Examples of the antiseptic include benzalkonium chloride, paraoxybenzoic acid, and chlorobutanol.

The dosage form of the formulation is not particularly limited, either. The pharmaceutical composition or quasi drug composition of the present invention may be in the form of, for example, tablet, powder, fine grain, granule, capsule, syrup, injection, external preparation, or suppository.

The pharmaceutical composition and quasi drug composition of the present invention are administered to the subject orally or parenterally (for example, intravenous, intraarterial, hypodermic, muscle, or intraperitoneal injection, transdermal, nasotracheal, or transmucosal administration, or application), depending on the dosage form or shape.

The term "subject" is not particularly limited, and examples thereof include human and mammals other than human (for example, pet animals, livestock, and experimental animals, and specific examples include mice, rats, guinea pigs, hamsters, monkeys, bovines, pigs, goats, sheep, dogs, cats, chickens, and partridges). In a preferred embodiment, the subject is human.

Additional aspects of the present invention relate to a product for topical use or topical use product, comprising the solubilized eggshell membrane products of the invention and to a method to produce the product for topical use, wherein preparation of the solubilized eggshell membrane products comprises the steps of (i) hydrolysation of eggshell membrane in the presence of the cysteine protease and L-cysteine HCl Anhydrous, (ii) filtration step based on phase separation and use of ion exchange resins, (iii) concentration step including vacuum evaporation system and (iv) drying step based on freeze-drying.

Yet an additional aspect of the present invention relates to a product for oral use or oral use product comprising the solubilized eggshell membrane products of the invention, as well as to a method to produce the product for oral use product, wherein preparation of the solubilized eggshell membrane products comprises the steps of (i) hydrolysation of eggshell membrane in the presence of the cysteine protease and L-cysteine HCl Anhydrous, (ii) filtration step based on phase separation and use of ion exchange resins, (iii) concentration with nanofiltration membranes and (iv) drying step based on spray drying.

The term "product for topical use" or "topical use product" includes but is not limited to creams, serums, lotions, shampoos, conditioners, nail polish.

The term "product for oral use" or "oral use product" refers to supplements or functional foods, as drinkable or edible products. These include but are not limited to fortified foods, food supplements, drinkable supplements, functional beverages, juices, etc.

The term "comprising" or "comprise" or "comprises" includes the term "consisting of" or "consist of" or "consists of".

Several preferred embodiments of the present invention will be described in greater detail below by means of specific examples.

### EXAMPLES

The following examples illustrate the present invention and are not to be understood as limiting the invention. In particular, the examples displayed correspond to compositions comprising papain, similar results having been obtained with other cysteine proteases such as bromelain, ficine, pepsin or protease K.

**Example 1:** Eggshell hydrolysis rates and organoleptic properties of the hydrolysis products obtained with compositions comprising papain and different reducing agents.

The reducing agents that were tested included: Metabisulfite (MBS), Non-leavening yeast, L-cysteine, N-acetyl Cysteine and L-cysteine HCl Anhydrous.

The hydrolysate powder obtained was characterised by measuring its purity (protein content) and the average size of the peptides present in the hydrolysate.

The sensory properties of both the hydrolysed product once lyophilised and a solution of this product prepared at a concentration of 15 mg/ml are analysed, as it is estimated that its expected use will be a solution at this concentration. To assess organoleptic characteristics, colour is assessed on a scale of 1 to 5, where 1 is dark brown, 2 light brown, 3 dark cream, 4 light cream and 5 white, and with regard to odour/flavour, 1 is very unpleasant, 2 unpleasant, 3 light, 4 very light and 5 neutral. Therefore, the higher the value obtained, the more desirable its sensory properties are.

The following tables 1A and 1B display the experimental parameters of the tests performed in Example 1, and results obtained therein. The table shows the average obtained after the products have been tested by 10 tasters in a blind test:

**Table 1A.**

| | TEST Nº | | | | | | | |
|---|---|---|---|---|---|---|---|---|
| | 1 | 2 | 3 | 4 | 5 | 6 | 7 | 8 |
| Eggshell membrane (g) | 2000 | 2000 | 2000 | 2000 | 2000 | 2000 | 2000 | 2000 |
| Water (I) | 20 | 20 | 20 | 20 | 20 | 20 | 20 | 20 |
| Papain (g) | 108 | 108 | 108 | 108 | 108 | 108 | 108 | 108 |
| MBS (g) | 120 | 120 | X | X | X | X | X | X |
| Non-leavening yeast (g) | X | X | 1200 | 1200 | 2000 | X | X | X |
| L-Cysteine (g) | X | X | X | X | X | 120 | 120 | 120 |
| L-Cysteine HCl Anhydrous (g) | X | X | X | X | X | X | X | X |
| N-acetyl Cysteine (g) | X | X | X | X | X | X | X | X |
| Hydrolysis time (h) | 17 | 17 | 17 | 17 | 17 | 17 | 17 | 17 |
| Hydrolysis temperature (ºC) | 55 | 55 | 55 | 55 | 55 | 55 | 55 | 55 |
| Hydrolysis pH | 7.06 | 7.03 | 7.05 | 7.09 | 7.01 | 7.02 | 7.02 | 7.07 |
| **Hydrolysis rate (%)** | **76.33** | **75.06** | **39.95** | **26.53** | **11.11** | **48.20** | **49.80** | **56.30** |
| Powder colour | 3.1 | | 2.7 | | | 4 | | |
| Powder smell | 2.3 | | 2.5 | | | 2.7 | | |
| Powder taste | 2.1 | | 2.1 | | | 2.7 | | |
| Solution colour | 2.9 | | 2.7 | | | 3.9 | | |
| Solution smell | 2.7 | | 2.5 | | | 2.8 | | |
| Solution taste | 2.7 | | 2.1 | | | 2.8 | | |
| **Organoleptic averge** | **2.6** | | **2.4** | | | **3.2** | | |

**Table 1B.**

| | TEST Nº | | | | | |
|---|---|---|---|---|---|---|
| | 9 | 10 | 11 | 12 | 13 | 14 |
| Eggshell membrane (g) | 2000 | 2000 | 2000 | 2000 | 2000 | 2000 |
| Water (I) | 20 | 20 | 20 | 20 | 20 | 20 |
| Papain (g) | 108 | 108 | 108 | 108 | 108 | 108 |
| MBS (g) | X | X | X | X | X | X |
| Non-leavening yeast (g) | X | X | X | X | X | X |
| L-Cysteine (g) | X | X | X | X | X | X |
| L-Cysteine HCl Anhydrous (g) | 120 | 120 | 120 | X | X | X |
| N-acetyl Cysteine (g) | X | X | X | 120 | 120 | 120 |
| Hydrolysis time (h) | 17 | 17 | 17 | 17 | 17 | 17 |
| Hydrolysis temperature (ºC) | 55 | 55 | 55 | 55 | 55 | 55 |
| Hydrolysis pH | 7.05 | 7.1 | 7.09 | 7.03 | 7.07 | 7.1 |
| **Hydrolysis rate (%)** | **75.25** | **75.44** | **73.70** | **51.33** | **56.89** | **59.92** |
| Powder colour | 4.3 | | | 4.1 | | |
| Powder smell | 3.3 | | | 2.9 | | |
| Powder taste | 3 | | | 2.8 | | |
| Solution colour | 4.1 | | | 4.0 | | |
| Solution smell | 3.5 | | | 3.1 | | |
| Solution taste | 3.2 | | | 3.1 | | |
| **Organoleptic average** | **3.6** | | | **3.3** | | |

As it can be observed, the best results are obtained with L-Cysteine HCl Anhydrous. A more efficient and complete hydrolysis is achieved with it, than with the other reducing agents, with the exception of MBS, the latter having the drawbacks of causing adverse reactions or allergies in sensitive individuals as well as negatively affecting the taste, aroma and colour of the final product.

In addition to the higher hydrolysis rate obtained with L-Cysteine HCl Anhydrous when compared with the other reducing agents (non-leavening yeast, L-cysteine and N-acetyl cysteine), facts such as sensory analysis of the products obtained also display a greater acceptance when L-Cysteine HCl Anhydrous is used as reducing agent.

Evaluation of peptides obtained when using these reducing agents also displays an improved quality and functionality of the peptides obtained when L-Cysteine HCl Anhydrous is used. The repeatability of the results obtained with L-Cysteine HCl Anhydrous has also been observed as being higher than with the other reducing agents.

Contrarily, non-leavening yeast displays lower efficiency, greater difficulty in controlling the enzymatic reactions, greater difficulty in handling and lower purity of the final product obtained, when compared with the other reducing agents tested.

In conclusion, the eggshell hydrolysis rate obtained with a composition of cysteine protease and L-Cysteine HCl Anhydrous as reducing agent, is higher and results in bioactive peptides, of better quality, and improved odour, colour and taste, than with compositions of different reducing agents.

**Example 2.** Performance of different compositions of Cysteine protease and L-Cysteine HCl Anhydrous at different concentrations.

The following example evaluates the concentration ranges at which compositions comprising Papain as Cysteine protease and L-Cysteine HCl Anhydrous display acceptable hydrolysis rates and result in products which are organoleptically acceptable or not.

The following tables display the tests performed in Example 2, and results obtained therein. The table shows the average obtained after the products have been tested by 10 tasters in a blind test:

**Table 2A.**

| | Papain 5,4 g/ 100 g eggshell membrane | | | | | |
|---|---|---|---|---|---|---|
| | 0.6g/ 100 g Mb* L-Cysteine HCl Anhydrous | 1.2g/ 100 g Mb L-Cysteine HCl Anhydrous | 1.5g/ 100 g Mb L-Cysteine HCl Anhydrous | 3 g/ 100 g Mb L-Cysteine HCl Anhydrous | 6 g/ 100 g Mb L-Cysteine HCl Anhydrous | 7,2 g/ 100 g Mb L-Cysteine HCl Anhydrous |
| Eggshell membrane (g) | 2000 | 2000 | 2000 | 2000 | 2000 | 2000 |
| Water (I) | 20 | 20 | 20 | 20 | 20 | 20 |
| Papain (g) | **108** | **108** | **108** | **108** | **108** | **108** |
| L-Cysteine HCl Anhydrous (g) | **12** | **24** | **30** | **60** | **120** | **144** |
| Hydrolysis time (h) | 17 | 17 | 17 | 17 | 17 | 17 |
| Hydrolysis temperature (ºC) | 45-65 | 45-65 | 45-65 | 45-65 | 45-65 | 45-65 |
| Hydrolysis pH | 7 | 7 | 7 | 7 | 7 | 7 |
| **Hydrolysis rate (%)** | **30,14** | **47,25** | **65,2** | **72,6** | **86,1** | **87,3** |
| **Organoleptic acceptance** | **YES** | **YES** | **YES** | **YES** | **YES** | **YES** |

| | | | | | | |
|---|---|---|---|---|---|---|
| *Mb: Eggshell Membrane | | | | | | |

**Table 2B.**

| Anhydrous | Papain 2.7 g/ 100 g eggshell membrane | | | |
|---|---|---|---|---|
| | 0.9 g/ 100 g Mb* L-Cysteine HCl Anhydrous | 3 g/ 100 g Mb L-Cysteine HCl Anhydrous | 6 g/ 100 g Mb L-Cysteine HCl Anhydrous | 7.2 g/ 100 g Mb L-Cysteine HCl Anhydrous |
| Eggshell membrane (g) | 2000 | 2000 | 2000 | 2000 |
| Water (I) | 20 | 20 | 20 | 20 |
| Papain (g) | **54** | **54** | **54** | **54** |
| L-Cysteine HCl Anhydrous | **18** | **60** | **120** | **144** |
| Hydrolysis time (h) | 17 | 17 | 17 | 17 |
| Hydrolysis temperature (ºC) | 55 | 55 | 55 | 55 |
| Hydrolysis pH | 7 | 7 | 7 | 7 |
| **Hydrolysis rate (%)** | **46%** | **50,36** | **79,3** | **78,7** |
| **Organoleptic acceptance** | **YES** | **YES** | **YES** | **YES** |

| | | | | |
|---|---|---|---|---|
| *Mb: Eggshell Membrane | | | | |

As observed in the test above, there are various combinations of concentrations of L-Cysteine HCl Anhydrous and papain that result in hydrolysis. Concentrations of L-Cysteine HCl Anhydrous higher than 10 g/ 100 g membrane sometimes raised acceptability issues with the participants, that were successfully overcome by addition of a flavour/ odour masking agent (Eg. 5-15 % Lemon; 10-15 % Grapefruit; 20-25 % Strawberry; 10-15 % Pineapple; 10-15 % Vanilla; 15-20 % Banana; 20-30% Raspberry; 25-50 % Cofee. This proved useful at any papain concentration in the range, and in particular at papain concentrations of 2.7 g/ 100 g membrane and 5.4 g/ 100 g membrane.

Papain at higher concentrations than 0.1 g/ 100 g eggshell membrane, with no concentration limit on the top, proved to provide adequate hydrolysis rates. Any acceptability issues that might be raised when using compositions with papain concentrations higher than 10 g/ 100 g eggshell membrane, were equally overcome by addition of a flavour/ odour masking agent, as those described above.

**Example 3:** Both eggshell and egg membrane previously separated from the shell, in the form of powder, were used as starting raw material for the hydrolysis tests of Example 3. All the reagent calculations were carried out assuming that there is 3% membrane in the shell. The results of hydrolysis rates and organoleptic conditions obtained for both starting raw materials are similar and are shown in the following table:

**Table 3.**

| | Test 1 | Test 2 | Test 3 | Test 4 |
|---|---|---|---|---|
| Eggshell membrane (g) | 2000 | 2000 | | |
| Eggshell (g) | | | 50000 | 50000 |
| Water (I) | 20 | 20 | 20 | 20 |
| Papain (g) | 108 | 108 | 55 | 55 |
| L-Cysteine HCl Anhydrous (g) | 120 | 120 | 60 | 60 |
| Hydrolysis time (h) | 17 | 17 | 17 | 17 |
| Hydrolysis temperature (ºC) | 55 | 55 | 55 | 55 |
| Hydrolysis pH | 7.05 | 7.1 | 7.12 | 7.08 |
| **Hydrolysis rate (%)** | **75.25** | **75.44** | **73.32*** | **71.32*** |
| Powder colour | 4.3 | | 3.6 | |
| Powder smell | 3.3 | | 3.3 | |
| Powder taste | 3 | | 2.7 | |
| Solution colour | 4.1 | | 3.2 | |
| Solution smell | 3.5 | | 3.5 | |
| Solution taste | 3.2 | | 2.8 | |
| **Organoleptic average** | **3.6** | | **3.2** | |

| | | | | |
|---|---|---|---|---|
| *For the calculation of the hydrolysis rate, the membrane content present in the eggshell is estimated to be 3%. | | | | |

The conclusion is that advantageous hydrolysis rates and organoleptically acceptable hydrolysis products are equally obtained with a composition comprising Papain and L-Cysteine HCl Anhydrous, both when the starting raw material is eggshell and egg membrane previously separated from the shell.

**Example 4:** Obtention of Eggshell membrane solubilisation product at an industrial scale, and evaluation through *in vitro* studies of its functional properties therein.

Eggshell membrane was subjected to a method comprising a hydrolysis phase, followed by a filtration phase, a concentration phase and a drying phase.

The hydrolysis at an industrial scale took place in a stainless steel tank, which included a heating system which set the temperature of the tank in the range of 35-80ºC, variable agitation system connected to a solids feed system, and a water inlet.

The initial hydrolysation step was followed by a filtration stage, wherein the filtration system included a phase separation stage and the use of the battery of ion exchange resins.

Next, a concentration system based on nanofiltration membranes was used, followed by vacuum evaporator; Finally drying was performed by means of a fluidised bed drying system.

The industrial process selected was the one that, at least theoretically, was more prone to alter the functionality of the peptides obtained to a greater degree.

The Eggshell membrane solubilisation product obtained by means of the method of above ("hydrolysate" in the examples below), was subjected to a series of *in vitro* studies with different cell lines, in order to determine the efficacy of the hydrolysis product against oxidation, healing, increased cell activity, new production of collagen, elastin and hyaluronic acid, collagenase and elastase inhibitory activity and finally anti-inflammatory activity.

These studies served to provide an exhaustive characterization and knowledge of the mechanism of action of the products obtained.

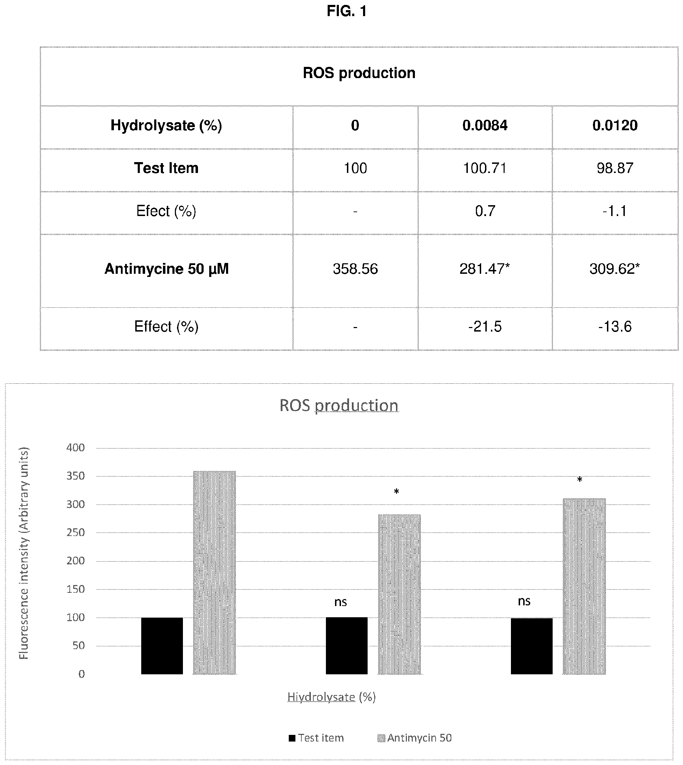

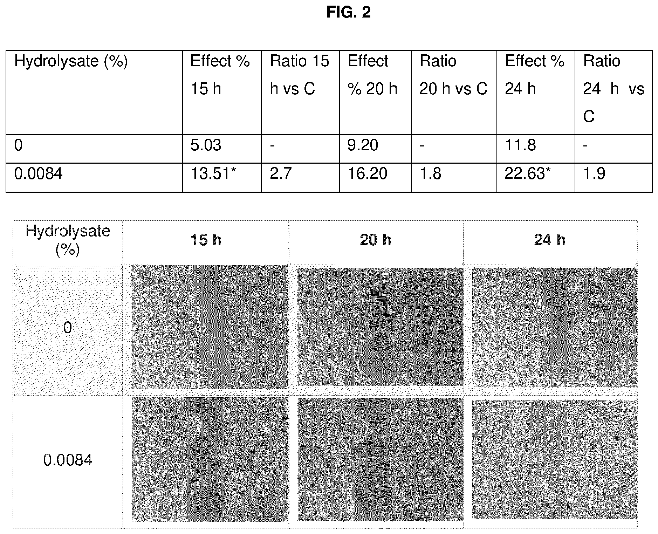

Some of the *in vitro* studies performed were as follows:
- ATP assay: The hydrolysate was tested in concentrations 0.025, 0.017, 0.012 and 0.0084%, on the human HeLa cell line *in vitro.* Although there was a two to fourfold dose-dependent ATP increase response, only the two highest concentrations showed a statistically significant difference regarding the control value. While the two lowest doses were not statistically significant, they were able to double the ATP production, which suggests an effect on cell activation. In contrast, the statistical increase induced by the highest doses are considered to impact on the initial phase of cell apoptosis.
- ROS (Reactive Oxygen Species) assay: The hydrolysate was tested in the concentration range of 0.0120-0.0084% in the same human HeLa cell line *in vitro.* The hydrolysate, at all concentrations tested, showed its ability to significantly reduce the ROS production stimulated by 50 µM Antimycin. Therefore, the hydrolysate may act as an antioxidant against putative marked stimulated oxidative processes. The results obtained with 0.0120% and 0.0084% are displayed in Figure 1.
- Wound healing assay: The hydrolysate was tested at the concentration of 0.0084% on human adult keratinocytes *in vitro.*
   The effect of the hydrolysate was tested in triplicate and the exposure time was approximately 24 hours. During the incubation period, images were taken at 0, 15, 20 and 24 hours.
   Under these test conditions, the hydrolysate showed a significant improvement of wound healing compared to the control. Results are displayed in Figure 2.
- Synthesis of procollagen and elastin. The hydrolysate was tested in concentrations 0.0058%, 0.0070% and 0.0084%, in Fibroblasts Balb/3T3 Clone A31 *in vitro.* The hydrolysate in the three concentrations tested proved to synthesize new procollagen, as well as new elastine.
- Synthesis of hyaluronic acid. The hydrolysate was assayed in 9 concentrations, 0.025, 0.021, 0.017, 0.014, 0.012, 0.010, 0.0084, 0.0070, 0.0058%, on Fibroblasts Balb/3T3 Clone A31 cell line *in vitro.* A dose-dependent increase in hyaluronic acid synthesis was observed throughout the whole range of concentrations tested, as compared to control basal values.
- Testing of collagenase and elastase. In the collagenase and elastase assay, the hydrolysate was tested at two concentrations of 0.0100 and 0.0120%, showing a statistically significant inhibition of about 3% for collagenase activity, and about 5% inhibition for elastase activity.
- Inflammatory biomarkers assay. C2C12 myoblasts and Opa1-deficient C2C12 cells were cultured and incubated with four different concentrations of hydrolysate: 0.025, 0.014, 0.012 and 0.00084. The following tests were performed: Measurement of concentrations of IL-1β; Analysis of gene expression of ASC, IL-6 and RAGE; DAF-FM Procedure for determination of Nitric Oxide (NO) in myoblasts. Main results are summarized as follows: In C2C12 Scr, hydrolysate at the concentration of 0.0084% induced an increase in IL-6. No other effects were observed in this cell line. In contrast, cells deficient in C2C12 Opa 1 clearly showed a marked increase in IL1 β, ASC, IL-6 and NO production compared to the normal homologous C2C12 Scr cell line, under these conditions, the hydrolysate was able to:
   - At 0.017% the test item decreased the IL1β levels, which is related to a putative anti-inflammatory activity, that could not be demonstrated as dose-dependent mechanism.
   - Furthermore, the test item was able to reduce the levels of ASC and NO at all concentrations.
   - RAGE expression was only reduced by the test item at the three lowest dose levels.
   - In contrast, the test item increased the IL-6 level only at the lowest dose.
      Consequently, it seems that the compound may have anti-inflammatory activity via certain routes.
      In conclusion, the hydrolysate proved useful to display anti-inflammatory capacity through certain pathways.

**Example 5:** Obtention of a cream formulated containing as the only active ingredient the eggshell membrane solubilization products of the present invention. The products were obtained industrially using the stages of (i) hydrolysation of eggshell membrane in the presence of papain and L-Cysteine HCl Anhydrous, (ii) phase separation and use of ion exchange resins as the filtration stage, (iii) vacuum evaporation system as the concentration system and (iv) freeze-drying as the drying system.

The cream was formulated for topical use, and contained different concentrations of the industrially obtained ingredient. In particular, three creams were formulated with different concentrations of industrial hydrolysate without perfume and another three with the same concentrations of hydrolysate as the former ones, but including perfume in their formulation.

**Table 4:**

| **Cream 1** | | |
|---|---|---|
| PHASE | % (W/W) | Function |
| A | c.s. 100 | Water |
| B | 12,00 | Emollient |
| | 5,00 | Emulsifier |
| C | 5,00 | Water |
| | 1,00 | Active ingredient |
| D | 1,50 | Preservative |
| | | |

| **Cream 2** | | |
|---|---|---|
| PHASE | % (W/W) | Function |
| A | c.s. 100 | Water |
| B | 12,00 | Emollient |
| | 5,00 | Emulsifier |
| C | 5,00 | Water |
| | 1,00 | Active ingredient |
| D | 1,50 | Preservative |
| | c.s. | Perfume |

| **Cream 3** | | |
|---|---|---|
| PHASE | % (W/W) | Function |
| A | c.s. 100 | Water |
| B | 12,00 | Emollient |
| | 5,00 | Emulsifier |
| C | 5,00 | Water |
| | 2,50 | Active ingredient |
| D | 1,50 | Preservative |

| **Cream 4** | | |
|---|---|---|
| PHASE | % (W/W) | Function |
| A | c.s. 100 | Water |
| B | 12,00 | Emollient |
| | 5,00 | Emulsifier |
| C | 5,00 | Water |
| | 2,50 | Active ingredient |
| D | 1,50 | Preservative |
| | c.s. | Perfume |

| **Cream 5** | | |
|---|---|---|
| PHASE | % (W/W) | Function |
| A | c.s. 100 | Water |
| B | 12,00 | Emollient |
| | 5,00 | Emulsifier |
| C | 5,00 | Water |
| | 5,00 | Active ingredient |
| D | 1,50 | Preservative |

| **Cream 6** | | |
|---|---|---|
| PHASE | % (W/W) | Function |
| A | c.s. 100 | Water |
| B | 12,00 | Emollient |
| | 5,00 | Emulsifier |
| C | 5,00 | Water |
| | 5,00 | Active ingredient |
| D | 1,50 | Preservative |
| | c.s. | Perfume |

Some examples of emollients used include: Isohexadecane, Ethyl Macacamiate, BR FOREST^{®} (Astrocaryum Murumuru Seed butter Cetearyl Alcohol (and) Glyceryl Stearate (and) Cetech-20 and Sodium), and combinations thereof. An example of emulsifier is POWDERFEEL WR^{®} (Polyacrylate (and) Hydroxypropiyl Starch Phosphate (and) Crambe Abyssinica Seed oil). An example of preservative used is Xylityl Sesquicaprylate.

To prepare the creams, phases A and B were heated at 45°C until completely dissolved, phase B was dispersed under stirring at 700 rpm for 10 minutes, then phase B was added on top of phase A under stirring and the mixture was kept under stirring for 30 minutes. The industrial hydrolysate was dissolved in water and added at room temperature over AB and finally the rest of the phases were added under stirring until complete incorporation.

To determine the functionality of the ingredient, an effectiveness study was carried out on 60 individuals, divided into three groups of 20 individuals, in which group A tested cream nº2, group B tested cream nº4 and group C tested cream nº6, for 60 days. The product was applied every night by applying a small amount of the cream on the forehead, nose, cheekbones and chin, after having previously carried out the usual facial cleansing. If any participant noticed any skin reaction during the 60 days, the treatment had to be stopped immediately. Finally, each individual had to fill in questionnaires regarding compliance of the cream tested.

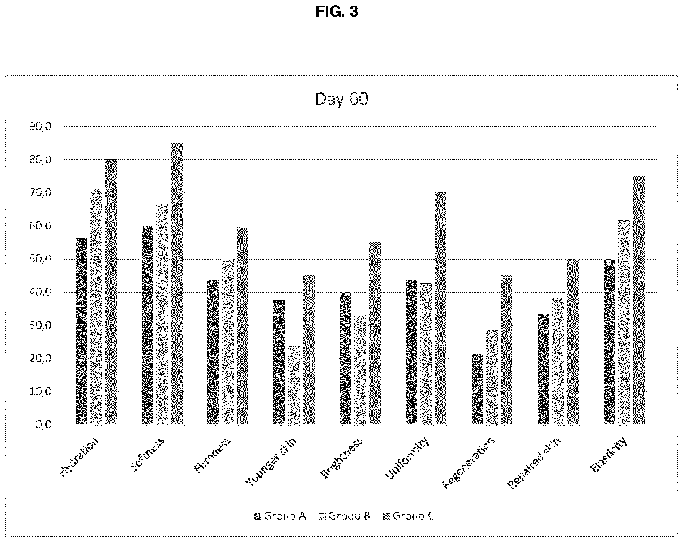

The results are as displayed in Figure 3.

For most of the parameters except for the smoothest skin, radiance and evenness, a very clear dose-dependent effect was observed, i.e. better results were obtained the higher the concentration of active ingredient in the cream. The parameters that clearly showed an improvement were hydration, smoothness, firmness, radiance, evenness and elasticity, in all of which more than 50% of the individuals who took part in the study observed benefits with the cream with the highest concentration. Notably, none of the participants who started the study had to discontinue it during the 60-day study due to adverse effects.

In addition to assessment of efficiency, the participants were enquired about the organoleptic properties of the tested cream.

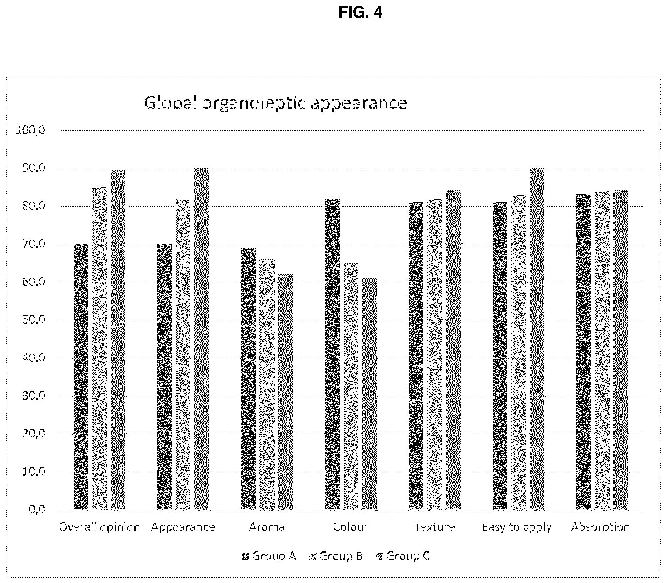

The results are displayed in Figure 4 and show that the smell and colour parameters are the least appreciated of the cream, although in both cases more than 60 percent of the participants indicated that they liked it at least moderately. On the other hand, regarding the rest of the properties, the higher the concentration of active ingredient, the more it was appreciated.

**Example 6:** Obtention of a drinkable product formulated containing as the only active ingredient the eggshell membrane solubilization products of the present invention. The products were obtained industrially using the stages of (i) eggshell hydrolysation in the presence of papain and L-Cysteine HCl Anhydrous, (ii) phase separation and use of ion exchange resins as the filtration phase, (iii) concentration with nanofiltration membranes and (iv) spray drying as drying system.

Preparation of the drinkable product: First, three dilutions were made in water of the industrially obtained ingredient at the following concentrations: 1.5, 3 and 15 mg/ml for blind tasting. In addition, two powdered products were formulated for subsequent use by pouring them into water at the time of use, each containing 300 mg of industrially obtained hydrolysate powder together with 300 mg of raspberry flavouring and 3 mg of sweetener, in the first case, and 300 mg of coffee flavouring, in the second case.

Organoleptic evaluation of the drinkable product: Each of the formulations was poured into 20, 100 and 200 ml of water before use so as to achieve final industrial hydrolysate concentrations of 1.5, 3 and 15 mg, respectively. A panel of 10 tasters analysed the sensory properties of each of the preparations.

The sensory evaluation of the drinkable product was very positive for all concentrations, achieving a media value of between 4 and 5 in a scale where 1 means very unpleasant, 2 means unpleasant, 3 means slight, 4 means very slight and 5 means neutral odour/taste. Additional evaluations were performed with the same drinkable products in the presence of flavourings such as raspberry or coffee. The latter flavour was the most preferred one.

Effectiveness of the drinkable product: A pilot study was designed to test the effectiveness of the drinkable coffee flavour product developed (Ovomet WS-coffee, egg membrane obtained by Eggnovo S.L. with coffee flavour).

Seventeen people over 18 years of age were selected, all of them diagnosed with osteoarthritis and having persistent knee pain with a rating of at least 2 pain points on the WOMAC scale (Western Ontario and Masters Universities). Participants agreed to replace any painkillers they may be taking with Ovomet WS-coffee after at least a 7-day washout period prior to the start of the new treatment. The pilot study lasted 20 days and participants completed the WOMAC questionnaire on days 0, 10 and 20. The WOMAC questionnaire consists of 5 questions related to pain, 2 questions related to joint stiffness and 17 questions related to joint functionality during physical activity.

The results are shown in Table 5 below, expressed as a percentage of supplemented individuals who showed improvements in the different parameters measured by the WOMAC questionnaire, after 10 and 20 days of treatment.

**Table 5.**

| | | % participants | |
|---|---|---|---|
| | | 10 days | 20 days |
| Pain reduction | 10% | 47 | 53 |
| | 20% | 18 | 35 |
| | 30% | 6 | 12 |
| Improved rigidity | 10% | 59 | 53 |
| | 20% | 41 | 41 |
| | 30% | 0 | 12 |
| | 40% | 0 | 12 |
| | 50% | 0 | 12 |
| Increased functionality | 5% | 47 | 65 |
| | 10% | 18 | 41 |
| | 20% | 6 | 24 |

These results show that the product tested after 10 days of treatment shows improvement in the symptoms of the participants, and this improvement increases as the treatment is prolonged. The absence of adverse effects described by the participants together with the results obtained indicate that the tested product maintains a high functionality and can be an effective treatment to improve the symptoms produced by osteoarthrosis and osteoarthritis.

## Claims

1. A composition for solubilizing eggshell membrane **characterized in that** it comprises a cysteine protease and L-cysteine HCl Anhydrous as reducing agent.

2. The composition of claim 1 wherein the cysteine protease is papain in a concentration of at least 0.1 g/ 100 g eggshell membrane, and the L-cysteine HCl Anhydrous concentration is in the range of 0.5 g/ 100 g eggshell membrane to 50 g/ 100 g eggshell membrane, for concentrations of papain and/ or L-cysteine HCl Anhydrous higher than 10 g/ 100 g eggshell membrane, a masking agent also being included.

3. The composition of claim 2, wherein the cysteine protease is papain in a concentration in the range of 0.1 g/ 100 g eggshell membrane to 50 g/ 100 g eggshell membrane, most preferably in the range of 0.1 g/ 100 g eggshell membrane to 10 g/ 100 g eggshell membrane, and even most preferably, in the range of 2.0 g/ 100 g eggshell membrane to 6.0 g/ 100 g eggshell membrane; and the L-cysteine HCl Anhydrous concentration is in the range of 0.5 g/ 100 g eggshell membrane to 50 g/ 100 g eggshell membrane, most preferably in the range of 0.5 g/ 100 g eggshell membrane to 10 g/ 100 g eggshell membrane.

4. Method for solubilizing eggshell membrane, comprising a hydrolysis phase wherein the eggshell membrane is treated with a composition according to any of claims 1 to 3.

5. The method of claim 4 wherein the hydrolysis phase is followed by a filtration phase, a concentration phase and a drying phase.

6. The method of any of claims 4 or 5, wherein the hydrolysis phase is performed at a temperature of between 30 and 80ºC, neutral pH and a reaction time of minimum 2 hours with no particular maximum limit.

7. The method of any of claims 5 or 6, wherein the filtration phase consists of subjecting the hydrolysate to one or more of the following processes: centrifugation and/or decantation and/or filtration through ion exchange resins.

8. The method of any of claims 5 to 7, wherein the concentration phase consists of evaporation and/or a concentration by nanofiltration membranes.

9. The method of any of claims 5 to 8, wherein the drying phase is performed by freeze-drying, spray drying or drying in a fluid bed, or a combination thereof.

10. The method of any of claims 4 to 9, wherein the source of egg membrane is eggshell or eggshell membrane previously separated from the shell.

11. Solubilized eggshell membrane products obtained by the eggshell membrane solubilization method according to any of claims 4 to 10.

12. A composition comprising the solubilized eggshell membrane products of claim 11.

13. Use of the composition according to claim 12 as a medicine, quasi drug, food, or cosmetic product, dispensed in the form of products for topical use, including creams, serums, and lotions, or in the form of products for oral use, including supplements or functional foods, as drinkable or edible products.

14. The use of claim 13 wherein the composition is used to improve skin, hair and nail health and/or gastrointestinal or joint condition or disease of a subject, in particular symptoms produced by osteoarthrosis and osteoarthritis.

15. A topical use product comprising the solubilized eggshell membrane products of claim 11.

16. Method to produce the topical use product of claim 15, wherein preparation of the solubilized eggshell membrane products comprises the steps of (i) hydrolysation of eggshell membrane in the presence of the cysteine protease and L-cysteine HCl Anhydrous, (ii) filtration step based on phase separation and use of ion exchange resins, (iii) concentration step including vacuum evaporation system and (iv) drying step based on freeze-drying.

17. A oral use product comprising the solubilized eggshell membrane products of claim 11.

18. Method to produce the oral use product of claim 17, wherein preparation of the solubilized eggshell membrane products comprises the steps of (i) hydrolysation of eggshell membrane in the presence of the cysteine protease and L-cysteine HCl Anhydrous, (ii) filtration step based on phase separation and use of ion exchange resins, (iii) concentration with nanofiltration membranes and (iv) drying step based on spray drying.
